# EUROPÄISCHE PATENTSCHRIFT

(11) **EP 0 970 717 B1**
(45) Veröffentlichungstag und Bekanntmachung des Hinweises auf die Patenterteilung: **02.07.2003**
(21) Anmeldenummer: 99113128.5
(22) Anmeldetag: 07.07.1999
(51) Int. Cl.: A61M 37/00, A61M 5/24, A61M 11/00

(54) **Medizinische Ultraschall-Injektionsspritze**
Medical ultrasonic injection syringe
Seringe d'injection ultrasonique médicale

(30) Priorität: 10.07.1998 DE 19830856
(43) Veröffentlichungstag der Anmeldung: 12.01.2000
(73) Patentinhaber: Boehm, Hans-Georg, Dr. rer. nat., D-61476 Kronberg/Ts (DE)
(72) Erfinder: Boehm, Hans-Georg, Dr. rer. nat., D-61476 Kronberg/Ts (DE)

(56) Entgegenhaltungen:
- WO-A-98/18391
- DE-A- 2 166 824
- FR-A- 2 190 176
- US-A- 4 169 984
- US-A- 5 437 606

## Beschreibung

### Stand der Technik :

In der Dentalmedizin sind Spritzengestelle für Glaskartuschen (Zylinderampullen) bspw. für Injektionen bekannt. Sie bestehen aus einem Metallgestell mit Fingerstütze, in das eine Kartusche eingeschoben wird. Beim Aufschrauben des Befestigungshütchens der Kanüle auf das Spritzengestell, durchstößt das hintere Ende der Kanüle die vordere Gummimembran der Kartusche. Zur Entleerung der Kartusche läßt sich dann deren hinterer Gummistopfen mit einer Kolbenstange vortreiben.

Eine piezoelektrische Vibration des gesamten Spritzengestells ist nach FR 2 190 176 A vom 25. Januar 1974 bekannt. Sie diente hier aber nur der Erhöhung der Viskosität des Kartuscheninhaltes, um das Spritzen in den Wurzelkanal von geöffneten Zähnen zu erleichtern und nicht zum Zerstäuben des Kartuschen-Inhalts.

### Problemlosung:

Ein wichtiger neuer Einsatz, um pharmakologische Lösungen der Humanmedizin nicht zu injizieren, sondern in kleinen Dosen als Nebel äußerlich auf eng begrenzte und schwer zugängliche Körperstellen (Zähne, Rachen, Nasenhöhle, Gehörgang etc.) fein und schonend verteilen zu können, ist dadurch erzielt, daß die aus der Kanüle herausgedrückte, minimale (weil kostbare) Wirkstoffmenge zur Applikation mit Ultraschall zerstäubt wird.

### Beschreibung:

Diese Aufgabe wird gemäß Anspruch 1 gelöst.

Dazu ist die leicht gebogene Kanülenspitze (1) durch den angekoppelten piezoelektrischen Ultraschallschwinger (2) in Resonanz gebracht, wodurch die herausgedrückten Flüssigkeitströpfchen fein zerstäubt werden. Dies ist dadurch erreicht, daß die ausschließlich vibrierenden Teile, also die Kanüle (1) mit ihrem Befestigungshütchen (4), dem Gewindeansatz der Spritze (5) mit angelötetem Piezo-Schwinger (2) und einem an dessen Unterseite angelötetem Metallzylinder (6) als Symmetriergewicht zur Definition des Schwingungsnullpunktes in der Masse klein, vom Spritzengestell (8) und der Kartusche (3) durch Lagerung in elastischen Puffern (7) abgekoppelt sind.

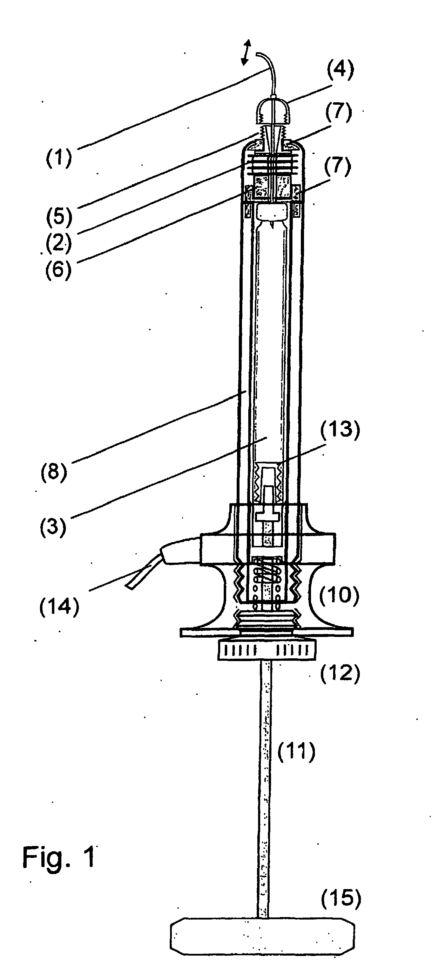

### Bezeichnungen in Fig. 1:

(1) Kanüle mit leicht gebogener, vibrierender Spitze
(2) Paket aus drei Piezoschwingern, Lötlaschen
(3) Glaskartusche mit vorderer Gummimembran durch die das hintere Ende der Kanüle (1) durchsticht und einem hinteren Gummistopfen, der mit einer Kolbenstange (11) zur Entleerung der Kartusche vorgetrieben wird
(4) Befestigungshütchen der Kanüle
(5) Gewindeansatz der Spritze, oben auf die Piezo-Schwinger gelötet
(6) Metall-Lochzylinder, definiert den Schwingungsnullpunkt, auf die Unterseite der Piezo-Schwinger gelötet
(7) Kautschukpuffer, lagern das Schwingersystem mit seiner angekoppelten Kanüle
(8) Spritzengestell
(10) Fingerstütze des Spritzengestells
(11) Kolbenstange zum Vortreiben des Gummistopfens (13)
(12) Öffnungsschraube am hinteren Teil des Spritzengestells (8) zum Nachladen einer neuen Kartusche
(13) hinterer Gummistopfen der Kartusche
(14) Anschluß des elektronischen Schwingkreises
(15) Handkrücke am Ende der Kolbenstange (11)

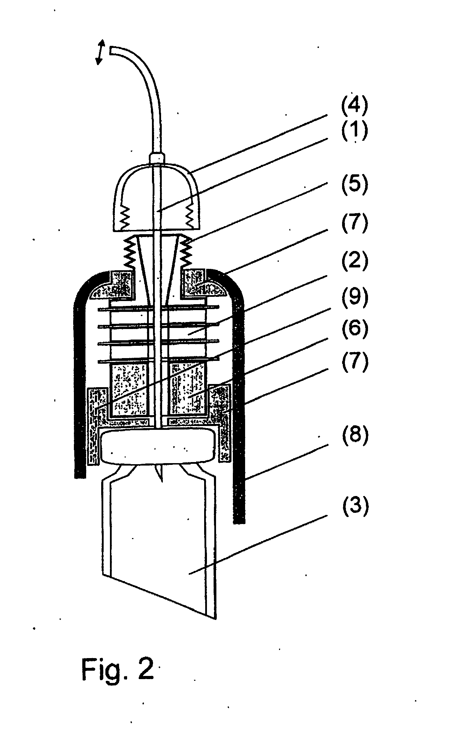

### Bezeichnungen in Fig. 2 :

(1) Kanüle mit leicht gebogener Spitze, die durch Piezo-Schwinger angeregt, in Resonanz schwingt
(2) Paket aus drei Piezoschwingern, Lötlaschen
(3) Glaskartusche mit vorderer Gummimembran
(4) Befestigungshütchen der Kanüle
(5) Gewindeansatz der Spritze, oben auf die Piezo-Schwinger gelötet
(6) Metall-Lochzylinder, definiert den Schwingungsnullpunkt, auf die Unterseite der Piezo-Schwinger gelötet
(7) Kautschukpuffer, lagern das Schwingersystem mit seiner angekoppelten Kanüle
(8) Spritzengestell
(9) Kerbe für die elektrische Schwingerzuleitung

## Patentansprüche

1. Medizinische Ultraschall-Injektionsspritze, bei welcher der von Hand herausgedrückte Kartuschen-Inhalt an der Spitze der Kanüle durch ihre Resonanzschwingung zu einem feinen Sprühnebel zerstäubt wird, bestehend aus einem Spritzengestell (8) mit Kanüle (1), einer Kartusche (3)' und einem Paket aus an den Piezo-Schwingern (2) oben angelötetem Gewindeansatz (5) und unten angelötetem Metall-Lochzylinder (6), welches in Kautschuk-Puffern (7) gelagert ist, sind **dadurch gekennzeichnet, daß** das Paket aus Gewindeansatz, Piezo-Schwingern und Metall-Lochzylinder elastisch im oberen Teil des Spritzengestells in Kautschuk-Puffern eingespannt ist.

## Claims

1. Medical ultrasonic injection syringe of which the content of its cartridge is expressed by hand and is sprayed as a fine mist out of the tip of the cannula by her resonance vibration, consisting of a syringe rack (8) with cannula (1), a cartridge (3) and a unit of a syringe thread (5) and a metal hole cylinder (6) which are soldered above or below on the piezo package (2), which is hold in rubber buffers (7), is **characterised by** the fact that the unity of the thread, the piezo package and the metal hole cylinder is edged elastically in rubber buffers at the upper part of the syringe rack.

## Revendications

1. Seringue d'injection ultrasonique médicale de laquelle le contenu de sa cartouche est exprimé à la main et est vaporisé comme un brouillard fin hors de la pointe de la canule par sa vibration de résonance, consiste d'un casier de seringue (8) avec canule (1), une cartouche (3) et une unité d'un pas de vis de la seringue (5) et d'un cylindre de métal avec trou (6) qui sont soudés au-dessus ou au-dessous du paquet des piezo cristaux (2) qui est inserée dans amortits de caoutchouc (7), est **caractérisée par le fait que** l'unité du pas de vis, des piezo cristaux et du cylindre de métal avec trou est encastrée élastiquement dans amortits de caoutchouc à la partie supérieure du casier de la seringue.
